# EUROPEAN PATENT SPECIFICATION

(11) **EP 2 098 249 B1**
(45) Date of publication and mention of the grant of the patent: **24.10.2012**
(21) Application number: 08152311.0
(22) Date of filing: 05.03.2008
(51) Int. Cl.: A61K 47/02, A61K 9/20, A61K 31/455

(54) **Nicorandil carriers with enhanced stability**
Nicorandilträger mit verbesserter Stabilität
Véhicules de nicorandil dotés d'une stabilité améliorée

(43) Date of publication of application: 09.09.2009
(73) Proprietor: Rivopharm SA, 6928 Manno (CH)
(72) Inventor: Poli, Piero, 6900, Lugano (CH); Carcano, Michela, 22026, Maslianico (IT)
(74) Representative: Chajmowicz, Marion

(56) References cited:
- EP-A- 0 230 932
- WO-A-01/98174
- WO-A-2006/045715
- WO-A-2008/014862

## Description

The present invention relates to nicorandil carrier with enhanced stability.

### BACKGROUND OF THE INVENTION:

Nicorandil is a coronary vasodilator endowed with a complex pharmacological activity, being both an organic nitrate and a potassium channel activator. It is used for the therapy of a number of cardiovascular diseases such as myocardial ischemia (especially angina pectoris) and congestive heart failure. The oral drugs containing pharmaceutical compositions of Nicorandil in a solid form suitable for repeated administrations are particularly used in therapy for preventing angina attacks.
As recognized in various patents (EP1001773, U.S. 4,822,808, W02006/016040), the pharmaceutical compositions of Nicorandil in a solid form are generally characterised by their unsatisfactory stability, especially in the presence of moisture and such other factors as acidity, temperature, light and oxygen.
Particular precautions are therefore required throughout their manufacturing process and during storage, to avoid the products' contact with moisture and to inhibit other degradation processes which may cause an appreciable reduction of the active ingredient content.
Ikorel® tablets, marketed by SANOFI, comprise nicorandil 10mg or 20mg, for the prevention and long term treatment of chronic stable angina pectoris.
Ikorel® tablets are presented in soft tempered aluminium foil/PVC blister strips of 10 tablets, in which each tablet is linked to a silica gel capsule desiccant.
The marketing authorisation specifies that the blister should be stored in a dry place below 25°C. Each blister strip should be used within 30 days of opening.

International patent application WO 2008/014862 describes a releasable container for the storage of moisture-sensitive medicaments having at least one inner wall containing at least one channel former and an absorbent. The releasable container described in WO 2008/014862 may contain at least one blister comprising pharmaceutical solid forms of a moisture-sensitive medicament.

There remains a need for nicorandil formulations or presentations with enhanced stability.

### SUMMARY OF THE INVENTION:

The inventors have now shown that replacing the desiccant of the Ikorel® blisters by a molecular sieve surprisingly improved stability of nicorandil.

According to the present invention, there is provided a carrier for nicorandil, in form of a blister pack, comprising one or several dose blister pockets each containing at least one tablet of nicorandil, and at least one blister pocket containing a molecular sieve.

### DETAILED DESCRIPTION OF THE INVENTION:

The carrier:
It is described carrier for nicorandil which may be any container into which nicorandil tablets can be stored such as a blister pack, a plastic, glass bottle or vial, or any suitable container. According to the invention, the carrier is a blister pack.

The blister pack:
Packs in blister pack form for the containment of a unit dose medicaments are envisaged, as are packs containing multiple unit dose blister pockets arranged sequentially or otherwise, such as in series form. A particular multi-unit dose arrangement comprises an elongate strip having multiple blister pockets arranged in series thereon.

The blister pack comprises a base sheet and a lid. The base sheet and lid may comprise the same or different materials.

For ease of manufacturing, and in order to provide the necessary properties to the packaging material, the blisters preferably comprise a nonthermoplastic substrate (such as a metal foil) and a heat sealable layer disposed thereon, and optionally an additional protective layer, such as a polymer film of polyester. The heat sealable layer is usually disposed on the inner surface of the assembled package. The additional protective layer is usually disposed on the surface opposite the heat sealable layer.

The substrate is preferably formed from aluminium foil. However, other metals for the substrate include, but are not limited to, tin, iron, zinc, or magnesium formed on a sheet by vacuum deposition or sputtering and a carboxyl group-containing polymer and/or copolymer layer formed on the metal layer by lamination.

In one aspect, the blister pack comprises a laminate. Suitably, the laminate comprises material selected from the group consisting of metal foil, organic polymeric material and paper. Suitable metal foils include aluminium or tin foil having a thickness of from 5 to 100µm, preferably from 10 to 50 µm, such as 20 to 30 µm. Suitable organic polymeric materials include polyethylene, polypropylene, polyvinyl chloride, polychlorotrifluoroethylene, polyethylene terephthalate and combinations thereof.

The heat sealable layer can be formed from any thermoplastic or thermosetting material such as a metal foil, an ionomer resin, polyolefin, or cycloolefin copolymer.

In a preferred embodiment, both the heat sealable layer and the thermoplastic substrate are metal foils, e.g. aluminium foils.

The outer protective layer, if present, can be formed of any material as long as the final laminate has the requisite properties.

Adhesives may be used to join the respective layers of materials together. The adhesive layers are typically substantially smaller in thickness relative to the thickness of the substrate, heat sealable and/or protective layers which they bond.

In a preferred embodiment, the carrier in form of a blister pack comprises 10 or less dose blister pockets each containing one tablet of nicorandil, and one blister pocket containing a molecular sieve. In a preferred embodiment, the blister pocket comprising the molecular sieve is located within a distance inferior to about 10cm, preferably inferior to about 8cm, from the blister pockets which contain nicorandil.

It is to be understood that the carrier, be it a blister pack or any other suitable container, does not comprise any silica gel.

### The molecular sieve :

With the appearance of small opaque pinkish beads, molecular sieves are generally synthetically produced. The molecular sieve material used in the present invention is preferably a metal-alumino silicates or a synthetic polymer gel. Preferred materials include hydroxyapatite, faujasite, calcium silicate, zirconia, zeolite, or the like. Exemplary synthetic polymers include, but are not limited to, stylene-divinylbenzene copolymer, cross-linked polyvinyl alcohol, cross-linked polyacrylate, cross-linked vinyl ether-maleic anhydride copolymer, cross-linked stylene-maleic anhydride copolymer or cross-linked polyamide, and combinations thereof.

In a preferred embodiment, the molecular sieve consists in sodium aluminosilicate.

Molecular sieves have many internal cavities that are linked by window openings of precise diameters. These diameters (measured in Ångstroms) classify molecular sieves - 3Å, 4Å, 5Å, and 10Å (also known as 13X).

Molecular sieves differ from conventional desiccants in the size of these pore openings. While conventional desiccants have a variety of pore size openings, the pore size opening of molecular sieves are all the same size - a "sieve" on the molecular scale. This type of structure enables molecular sieves to screen or select the components which will be adsorbed; for example, adsorption of water while excluding adsorption of valuable organics which might be part of a product's make-up (e.g. perfumes, plasticizers, solvents, etc.)
Adsorption occurs only of molecules with smaller diameters than these cavity openings. Larger molecules are excluded from adsorption. Preferentially adsorbed are molecules of greater polarity.
Molecular sieves adsorb water molecules and other contaminants from liquids and gases down to very low levels - often just 1 part per million.

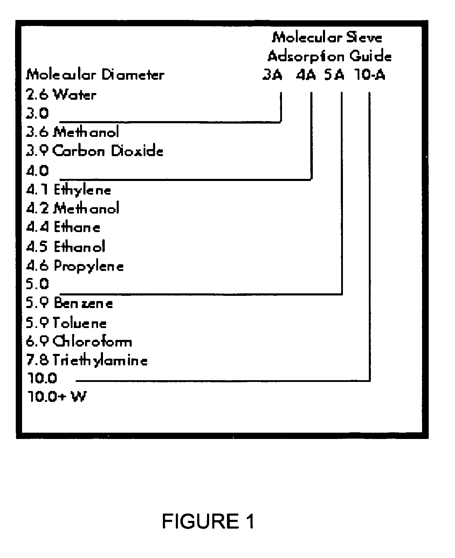

Figure 1 is a chart that is useful as a guide for the selection of molecular sieves.
A Molecular Sieve from 3Å to 8Å, preferably 4Å, is preferred in the present invention.

In a particular embodiment of the invention, the molecular sieve may be a Molecular Sieve 4A as provided by CSP technologies, which is made of sodium aluminosilicate, also called synthetic zeolite, in powder form. The formula of the zeolite is Na₂*Al₂O₃*2SiO₂*₂H₂O. Molecular Sieve 4A is protected in a polypropylene resin with an overall width of about 8,2mm, an overall length of about 17,7mm, an overall thickness of about 2,5mm, and shows an absorption capacity of at least 0,0615g at 22°C 80% relative humidity, a capacity over 24 hours of at least 0,06g at 22°C 80% relative humidity, and a saturation time of 150 to 300hours at 22°C 80% relative humidity.

The Figures and examples illustrate the invention without limiting its scope.

### LEGENDS TO THE FIGURES:

**Figure 1** is a chart that is useful as a guide for the selection of molecular sieves.
**Figures 2A and 2B** are graphs showing the percentage of impurities (rrt 0.14 for Figure 2A, rrt 0.25 for Figure 2B), at 25°C, 60%RH for batch 043/07.
**Figures 3A and 3B** are graphs showing the percentage of impurities (rrt 0.14 for Figure 3A, rrt 0.25 for Figure 3B), at 25°C, 60%RH for batch 044/07.
**Figures 4A and 4B** are graphs showing the percentage of impurities (rrt 0.14 for Figure 4A, rrt 0.25 for Figure 4B), at 30°C, 65%RH for batch 043/07.
**Figures 5A and 5B** are graphs showing the percentage of impurities (rrt 0.14 for Figure 5A, rrt 0.25 for Figure 5B), at 30°C, 65%RH for batch 044/07.
**Figures 6A and 6B** are graphs showing the percentage of impurities (rrt 0.14 for Figure 6A, rrt 0.25 for Figure 6B), at 40°C, 75%RH for batch 043/07.
**Figures 7A and 7B** are graphs showing the percentage of impurities (rrt 0.14 for Figure 7A, rrt 0.25 for Figure 7B), at 40°C, 75%RH for batch 044/07.

>

### EXAMPLES: Stability tests:

Two batches of Nicorandil tablets, 10mg (batch 043/07) and 20mg (batch 044/07) respectively, were prepared and packed according to the invention, in alu/alu blister together with a desiccant system.
Silica gel and molecular sieves were compared as desiccant systems and among molecular sieves, systems with different capacity of water absorption were considered.
043/07A: silica gel system
043/07B: 1 lozenge of desiccant plastic composition (Molecular Sieve 4A as provided by CSP technologies), containing molecular sieve 4Å, a base polymer of polypropylene and an elastomer (moisture absorbed in mg: about 64)
043/07C: 5 lozenges of desiccant plastic composition (Molecular Sieve 4A as provided by CSP technologies), containing molecular sieve 4Å, a base polymer of polypropylene and an elastomer (moisture absorbed in mg: about 320)

The blisters were placed on stability at the following conditions for 6 months:
25±2°C / 60±5% relative humidity (RH)
30±2°C / 65±5%RH
40±2°C / 75±5%RH

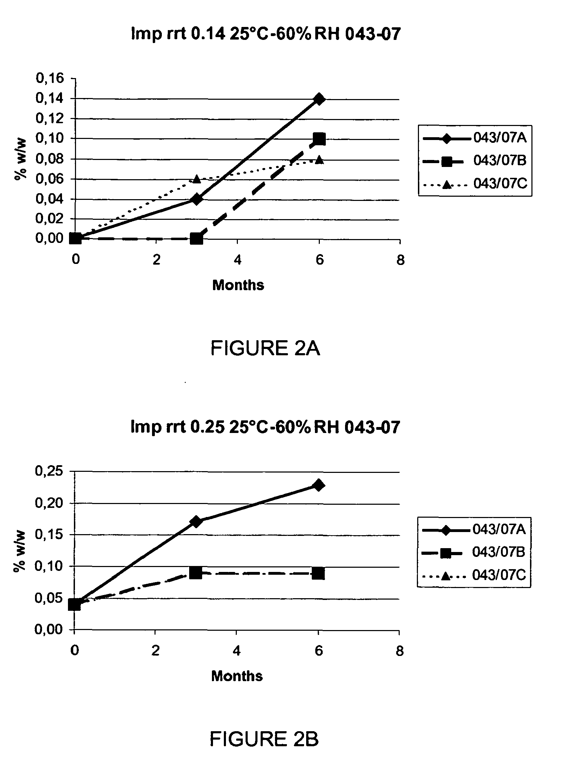

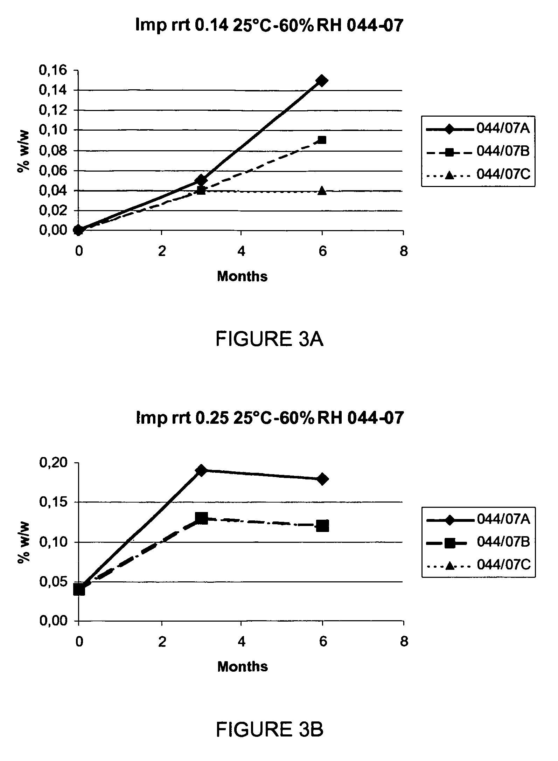

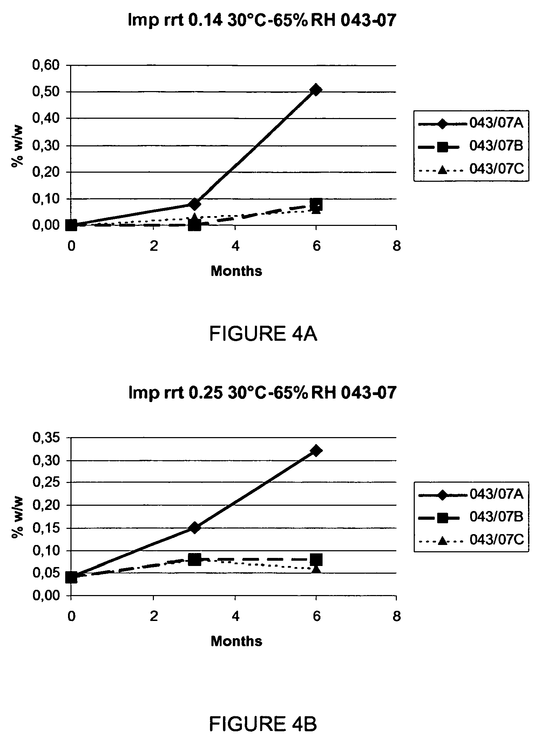

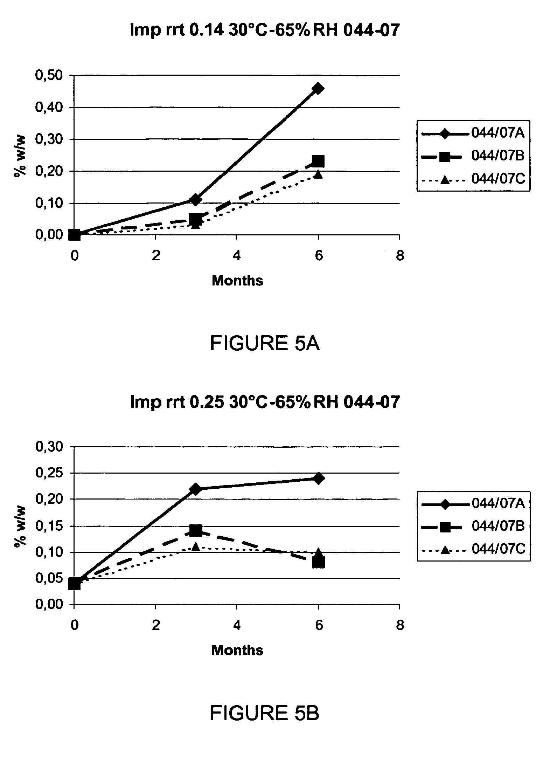

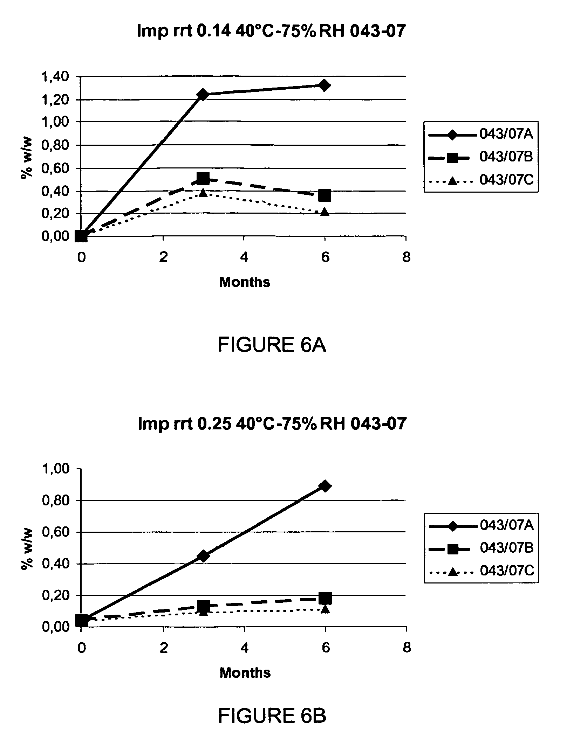

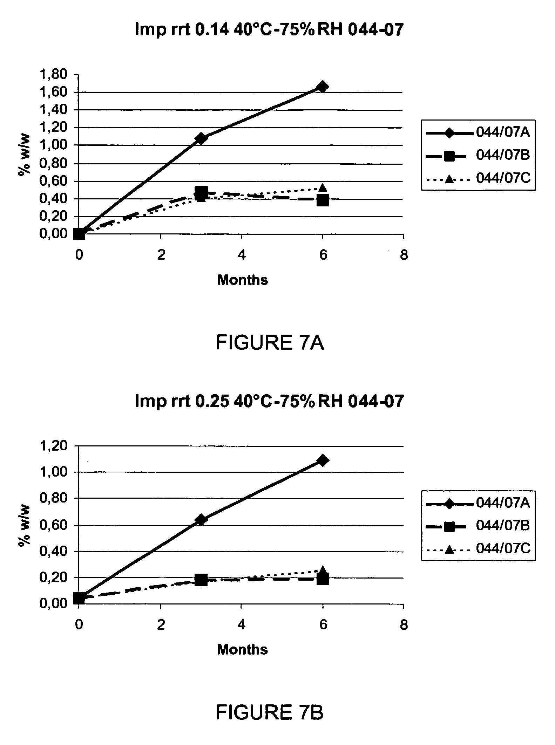

Nicorandil is particularly sensitive to humidity. Two impurities (named rrt0.14 and rrt0.25) are generated when the product is exposed to moisture and therefore were monitored during stability.
As shown on Figures 2 to 7, the level of the impurities is lower when the tablets are stored with the molecular sieves.

Nicorandil is thus more stable when stored in combination with the molecular sieves, as in the blister pack of the invention.

## Claims

1. A carrier for nicorandil which is in form of a blister pack, comprising :
- one or several dose blister pockets each containing at least one tablet of nicorandil, and
- at least one blister pocket containing a molecular sieve.

2. The carrier of claim 1, which comprises several dose blister pockets containing nicorandil, and one blister pocket containing a molecular sieve.

3. The carrier of any of claims 1 to 2, wherein the molecular sieve has window openings from 3Å to 8Å diameter.

4. The carrier of claim 3, wherein the molecular sieve has window openings of 4Å diameter.

5. The carrier of any of claims 1 to 4, wherein the molecular sieve is selected from the group consisting of metal-alumino silicates, synthetic polymer gels, hydroxyapatite, faujasite, calcium silicate, zirconia and zeolite.

6. The carrier of claim 5 wherein the molecular sieve is a metal-alumino silicate.

7. The carrier of claim 6, wherein the molecular sieve consists in sodium aluminosilicate.

8. The carrier of any of claims 1 to 7, wherein the molecular sieve lies within a thermoplastic container.

9. The carrier of claim 8, wherein the thermoplastic container is constructed from polypropylene.

10. The carrier of any of claims 1 to 9, which is a blister pack comprising at least one heat-sealable layer and at least one layer of a metal foil.

11. The carrier of claim 10, wherein the layers are made of metal foils selected from the group consisting of aluminium, tin, iron, zinc and magnesium.

## Patentansprüche

1. Ein Nicorandilträger, der die Form einer Blisterpackung hat, umfassend:
- eine oder mehrere Dosierungsblisterfächer, die mindestens eine Nicorandiltablette enthalten, und
- mindestens ein Blisterfach, das ein molekulares Sieb enthält.

2. Der Träger nach Anspruch 1, der mehrere Dosierungsblisterfächer umfasst, die Nicorandil enthalten, und ein Blisterfach, das ein molekulares Sieb enthält.

3. Der Träger nach einem der Ansprüche 1 bis 2, wobei das molekulare Sieb Fensteröffnungen mit einem Durchmesser von 3Å bis 8Å aufweist.

4. Der Träger nach Anspruch 3, wobei das molekulare Sieb Fensteröffnungen mit einem Durchmesser von 4Å aufweist.

5. Der Träger nach einem der Ansprüche 1 bis 4, wobei das molekulare Sieb ausgewählt ist aus der Gruppe bestehend aus Metall-Aluminiumsilikaten, synthetischen Polymergelen, Hydroxyapatit, Faujasit, Kalziumsilikat, Zirkonoxid und Zeolith.

6. Der Träger nach Anspruch 5, wobei das molekulare Sieb ein Metall-Aluminiumsilikat ist.

7. Der Träger nach Anspruch 6, wobei das molekulare Sieb aus Natriumaluminiumsilikat besteht.

8. Der Träger nach einem der Ansprüche 1 bis 7, wobei das molekulare Sieb innerhalb eines thermoplastischen Behälters liegt.

9. Der Träger nach Anspruch 8, wobei der thermoplastische Behälter aus Polypropylen hergestellt ist.

10. Der Träger nach einem der Ansprüche 1 bis 9, der eine Blisterpackung ist, die mindestens eine heißsiegelbare Schicht und mindestens eine Metallfolienschicht umfasst.

11. Der Träger nach Anspruch 10, wobei die Schichten aus Metallfolien gemacht sind, die aus der Gruppe ausgewählt sind bestehend aus Aluminium, Zinn, Eisen, Zink und Magnesium.

## Revendications

1. Support pour nicorandil sous la forme d'une plaquette, comprenant :
- une ou plusieurs poches de dose de plaquette, chacune contenant au moins un comprimé de nicorandil, et
- au moins une poche de plaquette contenant un tamis moléculaire.

2. Support selon la revendication 1, comprenant plusieurs poches de dose de plaquette contenant du nicorandil, et une poche de plaquette contenant un tamis moléculaire.

3. Support selon l'une quelconque des revendications 1 à 2, dans lequel le tamis moléculaire a des ouvertures de pore allant de 3Å à 8Å de diamètre.

4. Support selon la revendication 3, dans lequel le tamis moléculaire a des ouvertures de pore de 4Å de diamètre.

5. Support selon l'une quelconque des revendications 1 à 4, dans lequel le tamis moléculaire est choisi parmi le groupe constitué par les aluminosilicates de métal, les gels polymériques synthétiques, une hydroxyapatite, une faujasite, un silicate de calcium, un zircone et une zéolithe.

6. Support selon la revendication 5 dans lequel le tamis moléculaire est un aluminosilicate de métal.

7. Support selon la revendication 6 dans lequel le tamis moléculaire est un aluminosilicate de sodium.

8. Support selon l'une quelconque des revendications 1 à 7, dans lequel le tamis moléculaire est contenu dans un conteneur thermoplastique.

9. Support selon la revendication 8, dans lequel le conteneur thermoplastique est construit à partir de polypropylène.

10. Support selon l'une quelconque des revendications 1 à 9, ledit support étant une plaquette comprenant au moins une couche thermoscellable et au moins une couche en feuille de métal.

11. Support selon la revendication 10, dans lequel les couches sont faites de feuilles de métal choisi parmi le groupe constitué par l'aluminium, l'étain, le fer, le zinc et le magnésium.
